# EUROPEAN PATENT APPLICATION

(11) **EP 2 564 768 A1**
(43) Date of publication of application: **06.03.2013**
(21) Application number: 12194288.2
(22) Date of filing: 11.08.2004
(51) Int. Cl.: A61B 5/00

(54) **Method of constructing a biosensor**

(30) Priority: 14.08.2003 US 640980
(62) Divisional of application: 04780742.5
(71) Applicant: Isense Corporation, Wilsonville, OR 97070 (US)
(72) Inventor: Jansen, Lawrence B., Portland, OR Oregon 97219 (US); Ward, Kenneth D., Portland, OR Oregon 97231 (US); Anderson, Ellen M., Tualatin, OR Oregon 97062 (US)
(74) Representative: Moore, Barry

(57) **Abstract**

A method of creating an analyte sensor. The method starts with the step of providing an electrochemically active surface. Then, at least one nub made of dielectric material and extending transversely outwardly from the electrochemically active surface is created. A curable liquid is applied to the electrochemically active surface and the nub and is then cured. In this process, the nub, which could be one of several nubs, serves to support the liquid before and during the curing.

## Description

### FIELD OF THE INVENTION

The invention is generally related to the field of percutaneous analyte sensors.

### BACKGROUND ART

In the design and manufacture of an indwelling glucose sensor, a problem has been encountered in the application of viscous liquid layers of material, which are then cured, over the electrochemically active (platinum) surface. It is desirable to have an active surface area that is on the order of about a square millimeter. Unfortunately, when dip coating viscous liquids onto this relatively large area, it has been quite difficult to construct a coating having a thickness sufficient to produce an adequate response to the presence of glucose.

### DISCLOSURE OF THE INVENTION

In a first separate aspect, the present invention is an indwelling analyte sensor that has an active sensing region. This sensing region includes an electrochemically active surface and a membrane system that adheres to the electrochemically active surface. In addition, at least one nub of dielectric material extends outwardly from the electrochemically active surface and serves as a supportive structure to the membrane system.

In a second separate aspect, the present invention is a method of creating an analyte sensor. The method starts with the step of providing an electrochemically active surface. Then, at least one nub made of dielectric material and extending transversely outwardly from the electrochemically active surface is created. A curable liquid is applied to the electrochemically active surface and is then cured. In this process, the nub, which could be one of several nubs, serves to support the liquid before and during the curing.

The foregoing and other objectives, features and advantages of the invention will be more readily understood upon consideration of the following detailed description of the preferred embodiment(s), taken in conjunction with the accompanying drawings.

### BRIEF DESCRIPTION OF THE DRAWINGS

FIG. 1 is a side view of a work piece formed as part of the construction of a biosensor using the method of the present invention.
FIG. 2 is a side view of a sensor constructed from the work piece of FIG. 1.

### BEST MODE OF CARRYING OUT THE INVENTION

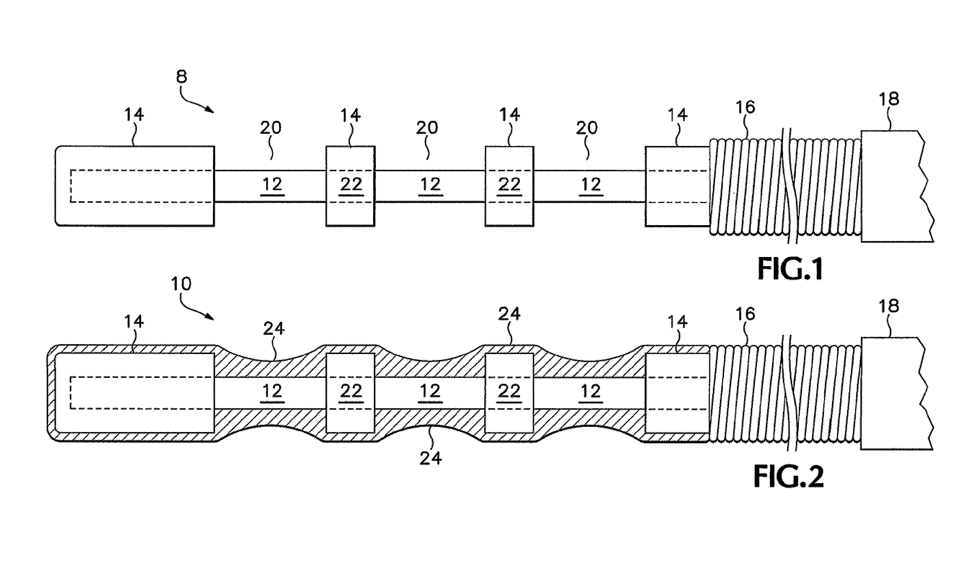

In a preferred embodiment of an analyte (typically glucose) sensor 10 (FIG.2) a 178 micron thick platinum wire 12 is coated with a 25 micron thick polyimide layer 14. A silver wire 16 is wrapped about a portion of layer 14. In addition, a stainless steel retractor lead 18 forms a portion of sensor 10.

Three cavities 20, each 2 mm long, are formed by laser ablating polyimide layer 14 to form a work piece 8 (FIG. 1). The polyimide between the cavities 20, forms a set of annular plates 22, that are supported by the adherence of the polyimide 14 onto wire 12. After this laser machining operation the work piece is ready to be dip coated with the material 24 that permits it to detect glucose. Typically, material 24 is comprised of a set of layers that are constructed through a sequence of dip coating operations interspersed with curing operations. These layers typically include an interferent excluding layer, a glucose oxidase layer and a permselective layer as described in U.S. Patent 5,165,407, which is hereby incorporated by reference as if fully set forth herein. The surface of each viscous fluid tends to form a shape somewhat like a catenary curve between plates 22. Accordingly a greater portion of viscous fluid adheres than would adhere without the presence of plates 22. This greater thickness, especially for glucose oxidase layer is very important in the creation of a robust response to the presence of glucose and oxygen.

### INDUSTRIAL APPLICABILITY

The invention generally finds industrial applicability in the production and providing of percutaneous analyte sensors.

The terms and expressions that have been employed in the foregoing specification are used as terms of description and not of limitation. There is no intention, in the use of such terms and expressions, of excluding equivalents of the features shown and described or portions thereof, it being recognized that the scope of the invention is defined and limited only by the claims which follow.

Furthermore, one or more aspects of the following numbered clauses may describe and relate to further aspects or features within the context of the present teaching:
1. An indwelling analyte sensor, comprising:
   (a) an active sensing region, including:
      (i) an electrochemically active surface; and
      (ii) a membrane system adhering to said electrochemically active surface;
   (b) at least one nub of dielectric material extending outwardly from said electrochemically active surface and serving as a supportive structure to said membrane system.
2. The sensor of clause 1, wherein said at least one nub is in the form of a plate.
3. The sensor of clause 1, wherein said electrochemically active surface is defined as part of a lengthwise body.
4. The sensor of clause 3, wherein said lengthwise body is circular in cross-section.
5. The sensor of clause 4, wherein said electrochemically active surface is circumferential to said circular lengthwise body.
6. The sensor of clause 5, wherein said nubs more specifically comprise annular plates.
7. The sensor of clause 2, wherein said nubs are displaced longitudinally from said electrochemically active surface.
8. The sensor of clause 2, wherein said membrane system includes multiple membranes.
9. The sensor of clause 2, wherein said membrane system includes an enzyme layer.
10. A method of creating an analyte sensor, comprising:
   (a) providing an electrochemically active surface;
   (b) creating at least one nub made of dielectric material and extending transversely outwardly from said electrochemically active surface;
   (c) applying a liquid to said electrochemically active surface and said at least one nub;
   (d) curing said liquid; and
   (e) whereby said at least one nub serves to support said liquid before and during said curing.
11. The method of clause 10, wherein said at least one nub is in the form of a plate.
12. The method of clause 10, wherein said electrochemically active surface is defined as part of a lengthwise body.
13. The method of clause 10, wherein said lengthwise body is circular in cross-section.
14. The method of clause 13, wherein said electrochemically active surface is circumferential to said circular lengthwise body.
15. The method of clause 14, wherein said nubs more specifically comprise annular plates.
16. The method of clause 10, wherein said nubs are displaced longitudinally from said electrochemically active surface.
17. The method of clause 10, wherein said membrane system includes multiple membranes.
18. The sensor of clause 10, wherein said membrane system includes an enzyme layer.
19. The sensor of clause 10 wherein said at least one nub is created by first providing a wire coated with dielectric material and then removing a portion of said dielectric material formed as a said nub.

## Claims

1. A method of creating an analyte sensor, comprising:
providing an electrochemically active surface;
forming at least one nub of dielectric material extending outwardly from the electrochemically active surface;
applying a liquid to, and substantially covering, the electrochemically active surface and the at least one nub; and
curing the liquid,
wherein the at least one nub supports the liquid before and during the curing.

2. The method of claim 1, wherein forming at least one nub comprises forming the at least one nub in the form of an annular plate.

3. The method of claim 1, wherein the electrochemically active surface is defined as part of a lengthwise body.

4. The method of claim 1, wherein the lengthwise body is circular in cross-section.

5. The method of claim 4, wherein the electrochemically active surface is circumferential to the lengthwise body.

6. The method of claim 1, wherein applying a liquid comprises applying a membrane system including multiple membrane layers to the electrochemically active surface and the at least one nub.

7. The method of claim 6, wherein the membrane system includes an enzyme layer.

8. The method of claim 7, wherein the enzyme comprises glucose oxidase.

9. The method of claim 1, wherein forming at least one nub of dielectric material extending outwardly from the electrochemically active surface comprises first providing the electrochemically active surface coated with dielectric material, and then removing a portion of the dielectric material to form at least one cavity and at least one nub.

10. The method of claim 9, wherein removing a portion of the dielectric material comprises laser ablating the dielectric material

11. The method of claim 1, wherein forming at least one nub of dielectric material comprises forming a plurality of nubs of dielectric material by first providing the electrochemically active surface coated with dielectric material, and then removing multiple portions of the dielectric material to form a plurality of cavities and a plurality of nubs.

12. The method of claim 1, wherein applying a liquid comprises dip coating the electrochemically active surface and the at least one nub.

13. The method of claim 12, wherein dip coating comprises multiple dip coating operations.
